**Europäisches Patentamt**

**European Patent Office**

**Office européen des brevets**

(11) Veröffentlichungsnummer: **0 421 023 B1**

## (12) EUROPÄISCHE PATENTSCHRIFT

(45) Veröffentlichungstag der Patentschrift: **15.03.95**

(21) Anmeldenummer: **89118628.0**

(22) Anmeldetag: **06.10.89**

(51) Int. Cl.⁶: **A61K 38/46**, A61K 38/48

---

(54) **Katabolische Enzyme zur Induktion des Tumornekrose-Faktors (TNF).**

---

(43) Veröffentlichungstag der Anmeldung:
**10.04.91 Patentblatt 91/15**

(45) Bekanntmachung des Hinweises auf die
Patenterteilung:
**15.03.95 Patentblatt 95/11**

(84) Benannte Vertragsstaaten:
**AT BE CH DE ES FR GB GR IT LI LU NL SE**

(56) Entgegenhaltungen:
**EP-A- 0 235 906**
**EP-A- 0 309 602**

**ROTE LISTE, 1987, Editio Cantor Aulen-
dorf/Württ, Zusammenfassungs-Nr. 85053,
"Wobe-Mugos"**

(73) Patentinhaber: **MUCOS EMULSIONSGESELL-
SCHAFT m.b.H.**
**Miraustrasse 17**
**D-13509 Berlin (DE)**

(72) Erfinder: **Ransberger, Karl**
**Gabriel-von-Seidl-Strasse 62**
**D-8022 Grünwald (DE)**
Erfinder: **Stauder, Gerhard, Dr.**
**Kathi-Kobus-Steig 1**
**D-8190 Wolfratshausen (DE)**

(74) Vertreter: **Patentanwälte Grünecker, Kinkeldey, Stockmair & Partner**
**Maximilianstrasse 58**
**D-80538 München (DE)**

**Beschreibung**

Die vorliegende Erfindung betrifft die Verwendung mindestens eines der Enzyme Pankratin, Bromelain, Papain, Lipase, Amylase, Trypsin und/oder Chymotrypsin für die Herstellung eines Arzneimittels zur Induktion von Tumornekrose-Faktor (TNF), ausgenommen Arzneimittel zur Behandlung von Tumoren und zur Bekämpfung von Virusinfektionen.

Tumornekrose-Faktor ist ein körpereigenes, im Monocyten-System gebildetes Glykoprotein vom MG 17 000 (Mensch) bis 150 000 (Maus). Ursprünglich wurde der Tumornekrose-Faktor als Protein isoliert, das in vitro und in vivo selektiv Tumorzellen abzutöten vermag. Diese Fähigkeit, Krebszellen selektiv anzugreifen ohne gesunde Zellen zu schädigen, unterstreicht das besondere Interesse an TNF als Krebstherapeutikum.

Inzwischen hat sich TNF zusätzlich als zentraler Immunmodulator erwiesen, der unter anderem bei Fibroblasten als Wachstumsfaktor, bei neutrophilen Granulozyten als Aktivator und bei der Differenzierung von Knochenmarkszellen-Lymphocyten als Inhibitor wirken kann. Tumornekrose-Faktor wird durch Immunmodulatoren, wie Interleukin-2, induziert. Interleukin-2 wird in einem aufwendigen und teuren Verfahren entweder aus T-Zellen isoliert oder mit Hilfe von gentechnologischen Methoden synthetisiert.

In der EP-A-0 309 602 wird die Verwendung von katabolischen Enzymen zur Behandlung der erworbenen Immunschwäche (AIDS) und deren Vorstadien (LAS und ARC), sowie der damit verbundenen Folgeerkrankungen, wie maligne Tumoren, beschrieben.

Aus der Roten Liste (Herausgeber: Bundesverband der Pharmazeutischen Industrie e.V., Frankfurt), 1987, ist die Anwendung des Präparats Wobe-Mugos, welches proteolytische Enzyme enthält, zur Behandlung von Tumoren und Viruserkrankungen bekannt.

Die EP-A-0 235 906 offenbart die Verwendung von TNF für die Herstellung eines Medikaments zur Behandlung oder Prophylaxe viraler Infektionen.

Der vorliegenden Erfindung liegt die Aufgabe zugrunde, einen wirksamen TNF-Induktor zur Verfügung zu stellen, der kostengünstig herstellbar ist.

Diese Aufgabe wird erfindungsgemäß dadurch gelöst, daß mindestens eines der Enyzme Pankreatin, Bromelain, Papain, Lipase, Amylase, Trypsin und/oder Chymotrypsin für die Herstellung eines Arzneimittels zur Induktion von Tumornekrose-Faktor verwendet wird, wobei Arzneimittel zur Behandlung von Tumoren und zur Bekämpfung von Virusinfektionen ausgenommen sind.

Es hat sich überraschenderweise gezeigt, daß die Enzyme Pankreatin, Bromelain, Papain, Lipase, Amylase, Trypsin und/oder Chymotrypsin die Synthese von Tumornekrose-Faktor in vitro und in vivo stimulieren können.

Die erfindungsgemäß verwendeten Enzyme lassen sich kostengünstig aus den folgenden Rohmaterialien isolieren.

Pankreatin wird aus Schweine- oder Rinderpankreas gewonnen.

Bromelain ist ein proteolytisch wirksames Enyzm aus dem Preßsaft der Ananas.

Papain ist ein proteolytisches Enzym, das aus dem Milchsaft der unreifen, fleischigen Früchte des Melonenbaums Carica papaya gewonnen wird.

Lipasen gehören zu der Untergruppe der Esterasen und werden aus Pankreas oder dem Pilz Rhizopus arrhizus gewonnen.

Amylasen sind Glykosid-spaltende Enzyme, die beispielsweise aus Pankreas oder speziellen Mikroorganismen isoliert werden.

Trypsin und Chymotrypsin sind proteolytische Enzyme, die ebenfalls im Pankreas gebildet werden und in Verbindung mit anderen Enzymen bereits therapeutisch eingesetzt wurden.

Vorzugsweise wird als Lipase Triacylglycerollipase und/oder als Amylase $\alpha$-Amylase verwendet. Diese zeigen eine gute Wirkung als TNF-Induktor.

Eine besondere Wirksamkeit zeigt sich bei Verwendung einer Kombination der Enzyme Pankreatin, Bromelain, Papain, Triacylglycerollipase, $\alpha$-Amylase, Trypsin und/oder Chymotrypsin. Neben der bemerkenswerten und unerwarteten Wirkung dieser Enzyme auf die TNF-Induktion hat die kombinierte Verwendung der genannten Enzyme weiterhin den Vorteil, daß auch bei einer Langzeitanwendung keine schädigenden Nebenwirkungen auftreten.

Weiterhin kann vorzugsweise zusätzlich Rutosid, ein zu den Flavonoiden gehörendes Glykosid, verwendet werden.

Eine besonders gute Wirksamkeit hat die kombinierte Verwendung von 50 - 200 mg, vorzugsweise 100 mg Pankreatin, 20 - 100 mg, vorzugsweise 45 mg Bromelain, 40 - 100 mg, vorzugsweise 60 mg Papa in, 5 - 50 mg, vorzugsweise 10 mg Triacylglycerollipase, 5 - 50 mg, vorzugsweise 10 mg $\alpha$-Amylase, 10 - 30 mg, vorzugsweise 24 mg Trypsin, 1 - 10 mg, vorzugsweise 1 mg Chymotrypsin und 10 - 100 mg, vorzugsweise 50 mg Rutosid x $3H_2O$ pro Dosiseinheit.

Weiterhin können die zu verwendenden Präparate zusätzlich Serratia-Peptidase enthalten.

Serratia-Peptidase kann aus einem Mikroorganismus der Gattung Serratia gewonnen werden.

Das zur Anwendung kommende Präparat kann weiterhin übliche Hilfs- und/oder Trägerstoffe enthalten.

Die Beispiele erläutern die Erfindung.

Beispiel 1:

Aus dem heparinisierten Blut gesunder Spender wurden über Lymphopreparation die mononuklearen Zellen (PMNC) isoliert. Die Trennung in adhärente (adh.) und nichtadhärente (nonadh.) Zellen erfolgte in mit FKS (fätales Kälberserum) vorbehandelten Petrischalen mit einer Inkubationszeit von 1,5 - 4 Stunden. Anschließend wurden diese Zellen in einer Konzentration von $5 \times 10^5$/ml auf 96-Vertiefungen Mikrotiterplatten (Costar) gebracht und mit Wobenzymlösung oder dessen Bestandteilen für 20 Stunden inkubiert (10 $\mu$g Enzym/ml).

Die überstände wurden abgehoben, vereinigt und bis zur Verwendung bei -20°C eingefroren. Die prozentuale Zytotoxizität der Überstände von Enzym-behandelten Zellen wurde im Vergleich zu Lipopolysaccharid-aktivierten Zellen mit Hilfe der Tumorzell-Linie L929 bestimmt (s. TNF-Bestimmung).

Die Ergebnisse sind in Tabelle 1 zusammengefaßt dargestellt.

**Tabelle 1** : Zytotoxizität (in %) der Überstände von enzymbehandelten peripheren mononuklearen Zellen (adhärent und nicht adhärent) im Vergleich zu lipopolysaccharid-aktivierten Zellen

| Versuch | Zellen | O | Wo[1] | Am[2] | Br[3] | Ch[4] | Li[5] | Pan[6] | Pap[7] | Tr[8] | LPS[10] |
|---------|--------|---|-----|-----|-----|-----|-----|------|------|-----|------|
| 51/56 | adh.[9] | 0 | 35 | 44 | 9 | 13 | 8 | 16 | 20 | 30 | 41 |
| 55/57 | adh. | 0 | 17 | 26 | 28 | 8 | 24 | 0 | 0 | 13 | 46 |
| 63/65 | adh. | 0 | 16 | 17 | 18 | 23 | 6 | 0 | 16 | 8 | 40 |
| 67/68 | adh. | 0 | 0 | 41 | 47 | 0 | 0 | 0 | 15 | 81 | 80 |
| 41/43 | adh. | 0 | 29 | 34 | 26 | 2 | 0 | 2 | 18 | 26 | 34 |
| 47/52 | adh. | 0 | 16 | 18 | n.d. | 8 | n.d. | n.d. | n.d. | n.d. | 36 |
| 63/65 | non adh. | 0 | 43 | 59 | 21 | 42 | 29 | 0 | 52 | 52 | 75 |
| 67/68 | non adh. | 0 | 18 | 47 | 39 | 0 | 23 | 0 | 9 | 50 | 82 |
| 67/68 | PMNC[11] | 0 | 19 | 50 | 70 | 0 | 0 | 0 | 65 | 0 | 81 |

[1] Wobenzym  [4] Chymotrypsin  [7] Papain  [10] Lipopolysacharid

[2] Amylase  [5] Lipase  [8] Trypsin  [11] periphere mononukleare Zellen

[3] Bromelain  [6] Pankreatin  [9] adhärente Zellen

Zum Nachweis von TNF in den überständen der Zellsuspensionen wurde die Zytotoxizität in Gegenwart von humanem Anti-TNF $\alpha$ in verschiedenen Verdünnungen untersucht. Hierfür wurden periphere mononukleare Zellen oder nur adhärente Zellen in einer Konzentration von $5 \times 10^5$/ml in 96-Vertiefungen Mikrotiterplatten zusammen mit PHA (Phytohaemagglutinin) (1 $\mu$g/ml), LPS (10 $\mu$g/ml) oder den Enzymen Wobenzym, Amylase, Bromelain, Pankreatin, Chymotrypsin oder Papain 24 Sunden bei 37°C inkubiert. Die überstände der Zellsuspensionen wurden auf 96-Vertiefungen Mikrotiterplatten übertragen, in deren Vertiefungen vorher L929-Zellen eingesetzt worden waren. Zusätzlich zu einer Actinomycin D-Lösung (10 $\mu$g/ml) kam humaner Anti-TNF $\alpha$ in verschiedenen Verdünnungen hinzu.

Die prozentuale Zytotoxizität wurde 20 Stunden später durch Anfärben der lebenden L929-Zellen mit Kristallviolett bestimmt (s. TNF-Bestimmung). Die Ergebnisse sind in Tabellen 2a, b und c dargestellt.

3

Tabelle 2a : Zytotoxizität (in %)

| Vorbehandlung der adh. Zellen | Verdünnung anti-TNF alpha | | | | |
|---|---|---|---|---|---|
| | 0 | 1:10000 | 1:1000 | 1:500 | 1:100 |
| PHA  1µg/ml | 73 | nd | 47 | nd | 22 |
| PHA  1µg/ml | 58 | nd | 15 | nd | 5 |
| LPS  10µg/ml | 55 | nd | 40 | nd | 0 |
| LPS  10µg/ml | 46 | nd | 0 | nd | 0 |
| LPS  10µg/ml | 61 | nd | 0 | nd | 0 |
| TNF  1000pg/ml | 74 | 66 | 39 | 34 | 33 |
| TNF  500pg/ml | 70 | 38 | 24 | 33 | 33 |
| TNF  250pg/ml | 62 | 26 | 20 | 0 | 0 |
| TNF  125pg/ml | 49 | 14 | 0 | 0 | 0 |

Tabelle 2b

| Zytotoxizität (in %) | | | |
|---|---|---|---|
| Vorbehandlung der adhärenten Zellen | Verdünnung anti-TNF alpha | | |
| | 0 | 1:1000 | 1:100 |
| Wobenzym 10µg/ml | 50 | 22 | 0 |
| Wobenzym 10µg/ml | 32 | 13 | 0 |
| Amylase 10µg/ml | 29 | 8 | 0 |
| Papain 40µg/ml | 58 | 30 | 8 |
| Papain 20µg/ml | 60 | 52 | 16 |
| Papain 5µg/ml | 42 | 24 | 0 |
| Papain 1µg/ml | 33 | 0 | 0 |

Tabelle 2c

| Zytotoxizität (in %) | | | |
|---|---|---|---|
| Vorbehandlung der PMNC | Verdünnung anti-TNF alpha | | |
| | 0 | 1:800 | 1:200 |
| Wobenzym | 80 | 15 | 0 |
| Amylase | 40 | 7 | 7 |
| Bromelain | 48 | 0 | 0 |
| Chymotrypsin | 19 | 7 | 0 |
| Lipase | 18 | 0 | 0 |
| Pankreatin | 22 | 8 | 0 |
| Trypsin | 50 | 0 | 0 |
| Papain | 58 | 0 | 0 |

Die Ergebnisse dieser Versuche zeigen, daß Wobenzym sowie dessen einzelne Bestandteile Pankreatin, Bromelain, Papain, Lipase, Amylase, Trypsin und Chymotrypsin in der Lage sind die Synthese von TNF in mononuklearen Blutzellen zu stimulieren.

Beispiel 2:

Um die synthetisierte TNF-Menge zu quantifizieren, wurden die folgenden Versuche durchgeführt:

$1 \times 10^6$/ml mononukleare Zellen aus dem Blut gesunder Spender wurden mit Wobenzym, Amylase, Papain und Bromelain (20 µg/ml) auf 24-Vertiefungen Mikrotiterplatten gebracht. Zu verschiedenen Zeitpunkten nach Inkubationsbeginn über einen Zeitraum von 24 Stunden wurden die überstände abgenommen und bis zur TNF-Bestimmung bei -20°C eingefroren. Die jeweiligen Ergebnisse der TNF-Bestimmung sind in den Abbildungen 1b bis e dargestellt. In Abbildung 1a sind die erhaltenen Einzel-Ergebnisse in einem Diagramm zusammengefaßt. Abbildung 2 zeigt die Kinetik der TNF-Synthese in den ersten fünf Stunden nach Inkubation mit dem jeweiligen Induktor.

## Abbildung 1a

**Abbildung 1b**

**Abbildung 1c**

6

**Abbildung 1d**

**Abbildung 1e**

Die Abbildungen 1a - e zeigen die TNF-Konzentration über einen Zeitraum von 24 h. Über den gesamten Untersuchungszeitraum kann für Wobenzym und seine Bestandteile TNF nachgewiesen werden. Besonders hohe Konzentrationen im Vergleich zu Lipopolysaccharid werden bei Verwendung der Enzyme Amylase und Bromelain erzielt.

## Abbildung 2

Die Abbildung 2 zeigt den Verlauf der TNF-Konzentration im Überstand von enzymbehandelten mononuklearen Blutzellen über einen Inkubationszeitraum von 5 h. Die höchsten TNF-Syntheseraten werden bei Induktion mit Lipopolysaccharid und dem Enzym Amylase, sehr gute Syntheseraten mit Wobenzym und Papain erzielt.

Weiterhin wurde die synthetisierte Menge an TNF in Abhängigkeit von der zur Induktion verwendeten Enzymmenge bestimmt. Hierfür wurden mononukleare Zellen aus dem Blut gesunder Spender einer Konzentration von 1 x 10$^6$/ml auf 24-Vertiefungen Mikrotiterplatten in Gegenwart von Wobenzym, Amylase, Bromelain oder Papain in den Konzentrationen 40 μg, 20 μg, 10 μg, 5 μg, 2 μg oder 1 ug pro ml für 7 Stunden inkubiert. Die Überstände wurden bei -20°C eingefroren und anschließend für die TNF-Bestimmung verwendet. Die jeweiligen Ergebnisse sind in den Abbildungen 3b bis e dargestellt. Eine Zusammenfassung der erhaltenen Einzel-Ergebnisse zeigt die Abbildung 3a.

**Abbildung 3a**

**Abbildung 3b**

**Abbildung 3c**

EP 0 421 023 B1

**Abbildung 3d**

Bromelain

**Abbildung 3e**

Papain

Die Abbildungen 3a - e zeigen, daß die gebildete Menge an TNF mit der Konzentration des zur Induktion verwendeten Enzymes ansteigt. Dabei ist die Konzentrationsabhängigkeit der Induktion wiederum von dem jeweils verwendeten Enzym oder der Enzymkonzentration abhängig.

11

TNF-Bestimmung:

L-929 (transformierte Mausfibroblastenlinie) in der Wachstumsphase (3. - 4. Tag nach Umsetzen) wurden mit Trypsin gelöst und 5 x $10^4$ /well auf 96-Vertiefungen Mikrotiterplatten aufgetragen. Nach 24 Stunden wurde der Überstand abgesaugt, 100 $\mu$l Actinomycin D (10 $\mu$g/ml) und 100 $\mu$l Probe pro well eingesetzt. Nach einer Inkubation von weiteren 24 Stunden wurde der Überstand erneut abgesaugt, wobei lebende Zellen adhärent bleiben. Diese werden mit Kristallviolett angefärbt und die Absorption bei 590 nm mittels einem Photometer bestimmt. Dabei ergibt sich prozentuale Zytotoxizität nach

$$\% \ Zytotoxit\ddot{a}t = \frac{Abs. \ (Kontrolle) - Abs. \ (Probe)}{Abs. \ (Kontrolle)} \times 100$$

Die Bestimmung der TNF-Menge erfolgte mit Hilfe einer Eichkurve, die mit rTNF (Genzym) erstellt wurde.

**Patentansprüche**

1. Verwendung mindestens eines der Enzyme Pankreatin, Bromelain, Papain, Lipase, Amylase, Trypsin und/oder Chymotrypsin für die Herstellung eines Arzneimittels zur Induktion von Tumornekrose-Faktor (TNF), ausgenommen Arzneimittel zur Behandlung von Tumoren und zur Bekämpfung von Virusinfektionen.

2. Verwendung mindestens eines der Enzyme Pankreatin, Bromelain, Papain, Lipase, Amylase, Trypsin und/oder Chymotrypsin zur in vitro-Induktion von Tumornekrose-Faktor (TNF).

3. Verwendung nach Anspruch 1 oder 2,
   **dadurch gekennzeichnet**,
   daß als Lipase Triacylglycerollipase verwendet wird.

4. Verwendung nach Anspruch 1 oder 2,
   **dadurch gekennzeichnet**,
   daß als Amylase $\alpha$-Amylase verwendet wird.

5. Verwendung nach Anspruch 1 oder 2,
   **dadurch gekennzeichnet**,
   daß eine Kombination der Enzyme Pankreatin, Bromelain, Papain, Triacylglycerollipase, $\alpha$-Amylase, Trypsin und/oder Chymotrypsin verwendet wird.

6. Verwendung nach mindestens einem der Ansprüche 1-5,
   **dadurch gekennzeichnet**,
   daß zusätzlich Rutosid verwendet wird.

7. Verwendung nach mindestens einem der Ansprüche 1-6
   **dadurch gekennzeichnet**,
   daß 50 - 200 mg, vorzugsweise 100 mg Pankreatin, 20 - 100 mg, vorzugsweise 45 mg Bromelain, 40 - 100 mg, vorzugsweise 60 mg Papain, 5 - 50 mg, vorzugsweise 10 mg Triacylglycerollipase, 5 - 50 mg, vorzugsweise 10 mg $\alpha$-Amylase, 10 - 30 mg, vorzugsweise 24 mg Trypsin, 1 - 10 mg, vorzugsweise 1 mg Chymotrypsin und 10 - 100 mg, vorzugsweise 50 mg Rutosid x 3$H_2$O pro Dosiseinheit verwendet werden.

8. Verwendung nach mindestens einem der Ansprüche 1-7,
   **dadurch gekennzeichnet**,
   daß zusätzlich Serratia-Peptidase verwendet wird.

## Claims

1. Use of at least one of the enzymes pancreatin, bromelain, papain, lipase, amylase, trypsin and/or chymotrypsin for the preparation of a drug for the induction of tumour necrosis factor (TNF), with the exception of drugs for the treatment of tumours and control of virus infections.

2. Use of at least one of the enzymes pancreatin, bromelain, papain, lipase, amylase, trypsin and/or chymotrypsin for the *in vitro* induction of tumour necrosis factor (TNF).

3. Use according to Claim 1 or 2, **characterized in that** triacylglycerol lipase is used as the lipase.

4. Use according to Claim 1 or 2, **characterized in that** $\alpha$-amylase is used as the amylase.

5. Use according to Claim 1 or 2, **characterized in that** a combination of the enzymes pancreatin, bromelain, papain, triacylglycerol lipase, $\alpha$-amylase, trypsin and/or chymotrypsin is used.

6. Use according to at least one of Claims 1-5, **characterized in that** rutoside is additionally used.

7. Use according to at least one of Claims 1-6, **characterized in that** 50 - 200 mg, preferably 100 mg, of pancreatin, 20 - 100 mg, preferably 45 mg, of bromelain, 40 - 100 mg, preferably 60 mg, of papain, 5 - 50 mg, preferably 10 mg, of triacylglycerol lipase, 5 - 50 mg, preferably 10 mg, of $\alpha$-amylase, 10 - 30 mg, preferably 24 mg, of trypsin, 1 - 10 mg, preferably 1 mg, of chymotrypsin and 10 - 100 mg, preferably 50 mg, of rutoside trihydrate are used per dosage unit.

8. Use according to at least one of Claims 1-7, **characterized in that** Serratia peptidase is additionally used.

## Revendications

1. Utilisation d'au moins l'une des enzymes pancréatine, bromélaïne, papaïne, lipase, amylase, trypsine et/ou chymotrypsine pour la préparation d'un médicament pour l'induction du facteur de nécrose tumorale (TNF), à l'exception des médicaments pour le traitement des tumeurs et pour lutter contre les infections virales.

2. Utilisation d'au moins l'une des enzymes pancréatine, bromélaïne, papaïne, lipase, amylase, trypsine et/ou chymotrypsine pour l'induction in vitro du facteur de nécrose tumorale (TNF).

3. Utilisation selon la revendication 1 ou 2, caractérisée en ce que l'on utilise, comme lipase, la triacylglycérollipase.

4. Utilisation selon la revendication 1 ou 2, caractérisée en ce que l'on utilise, comme amylase, l'$\alpha$-amylase.

5. Utilisation selon la revendication 1 ou 2, caractérisée en ce que l'on utilise une combinaison des enzymes pancréatine, bromélaïne, papaïne, triacylglycérollipase, $\alpha$-amylase, trypsine et/ou chymotrypsine.

6. Utilisation selon l'une au moins des revendications 1 à 5, caractérisée en ce que l'on utilise en plus du rutoside.

7. Utilisation selon l'une au moins des revendications 1 à 6, caractérisée en ce que l'on utilise 50 à 200 mg, de préférence 100 mg de pancréatine 20 à 100 mg, de préférence 45 mg de bromélaïne 40 à 100 mg, de préférence 60 mg de papaïne, 5 à 50 mg, de préférence 10 mg de triacylglycérollipase, 5 à 50 mg, de préférence 10 mg d' $\alpha$-amylase, 10 à 30 mg, de préférence 24 mg de trypsine, 1 à 10 mg, de préférence 1 mg de chymotrypsine et 10 à 100 mg, de préférence 50 mg de rutoside x 3 $H_2O$ par dose unitaire.

8. Utilisation selon l'une au moins des revendications 1 à 7, caractérisée en ce que l'on utilise en plus de la peptidase de Serratia.